# EUROPÄISCHE PATENTSCHRIFT

(11) **EP 1 309 284 B1**
(45) Veröffentlichungstag und Bekanntmachung des Hinweises auf die Patenterteilung: **17.05.2006**
(21) Anmeldenummer: 01923599.3
(22) Anmeldetag: 22.02.2001
(51) Int. Cl.: A61B 18/20

(54) **HANDSTÜCK ZUR ABSTRAHLUNG VON LICHT AUF EINE HAUTFLÄCHE**
HANDPIECE FOR RADIATING LIGHT ONTO A SKIN SURFACE DURING A MEDICAL OR COSMETIC SKIN TREATMENT
PIECE A MAIN DESTINEE AU RAYONNEMENT DE LUMIERE SUR UNE SURFACE CUTANEE LORS D'UN TRAITEMENT CUTANE MEDICAL OU COSMETIQUE

(30) Priorität: 23.02.2000 DE 10008977; 28.04.2000 DE 10021278
(43) Veröffentlichungstag der Anmeldung: 14.05.2003
(73) Patentinhaber: Carl Zeiss Meditec AG, 07745 Jena (DE)
(72) Erfinder: ELBRECHT, Jens, 07747 Jena (DE); GRIEGER, Jan, 07743 Jena (DE)
(74) Vertreter: Geyer, Fehners & Partner
(86) Internationale Anmeldenummer: PCT/EP2001/002014
(87) Internationale Veröffentlichungsnummer: WO 2001/062340

(56) Entgegenhaltungen:
- EP-A- 0 783 904
- WO-A-99/04707
- WO-A-99/46005
- US-A- 3 327 712
- US-A- 4 718 416
- US-A- 5 395 362
- US-A- 5 735 844
- US-A- 5 755 751
- US-A- 5 928 223
- US-A- 5 968 033

## Beschreibung

### Gebiet der Erfindung

Die Erfindung bezieht sich auf ein Handstück zur Abstrahlung von Licht auf eine Hautfläche bei einer medizinischen oder kosmetischen Hautbehandlung, wobei im Handstück ein optisches Koppelelement vorhanden ist, dessen Lichteintrittsfläche einer Lichtquelle zugewandt ist und dessen Lichtaustrittsfläche während der Hautbehandlung mit der Hautfläche in Kontakt steht.

Bei der Lichtquelle kann es sich sowohl um eine herkömmliche (thermische) Lichtquelle als auch um eine Laserstrahlungsquelle handeln. Das Koppelelement dient dabei im wesentlichen der Strahlformung, das heißt der Beeinflussung des auf die Haut zu richtenden Lichtes im Hinblick auf die geometrische Querschnittsform des Strahlenganges, die Ausdehnung der Querschnittsfläche und der Verteilung der Strahlungsintensität innerhalb des Strahlenganges beim Auftreffen auf die Haut.

### Stand der Technik

Für die verschiedenartigsten dermatologischen Behandlungen mit Licht sind im Stand der Technik unterschiedliche Handstücke bekannt geworden, die in Abhängigkeit von der jeweiligen Behandlung mit unterschiedlichen Mitteln zur Strahlformung ausgestattet sind. So sind beispielsweise Handstücke zur Ausübung der sogenannten Non-Kontakttechnik bekannt, wobei ein zumindest abschnittsweise frei in der Atmosphäre verlaufender Strahlengang auf die Haut appliziert wird. Der Strahlengang wird durch eine Optik geformt und mit der Optik auch die Intensitätsverteilung innerhalb des Strahlquerschnittes beeinflußt, beispielsweise homogenisiert. Die Optik weist eine das Licht abstrahlende Fläche auf, die während der Behandlung nicht mit der Haut in Kontakt steht; der gewünschte Fleckdurchmesser am Behandlungsort wird unter Ausnutzung der Strahlungsdivergenz durch Variation des Abstandes zwischen der abstrahlenden Fläche und der Hautfläche verändert.

Eine andere Verfahrensweise ist als Kontakttechnik bekannt, bei der ein transparentes optisches Medium, durch welches das Licht hindurchgeführt wird, mit seiner Lichtaustrittsfläche auf die Haut aufgesetzt wird. Durch das Aufsetzen der Lichtaustrittsfläche auf die Haut wird eine Indexanpassung zur Haut erreicht und insofern verhindert, daß ein Großteil der Energie von der Haut zurückgestreut wird und damit für die Applikation nicht zur Verfügung steht.

Ein solches Handstück ist über eine Strahlführungseinrichtung mit einer Laserstrahlungsquelle verbunden, wobei die Strahlführungseinrichtung so flexibel ist, das das Handstück relativ frei beweglich und so der Laserstrahl vom Operateur leicht auf das Behandlungsareal zu richten ist. Innerhalb des Handstückes ist der Strahlführungseinrichtung ein optisches Element, beispielsweise aus Quarz oder Saphir bestehend, nachgeordnet, das über eine Lichteintrittsfläche und eine Lichtaustrittsfläche, die auf die Haut aufzusetzen ist, verfügt.

Nachteilig hierbei ist vor allem der verhältnismäßig große Verlust an Lichtleistung bis zu 30%. Die dabei auftretende Verlustwärme führt zu Problemen, die, wenn überhaupt, nur durch aufwendige konstruktive Maßnahmen zu lösen sind, denn für dermatologische Behandlungen sind elektromagnetische Strahlungen mit verhältnismäßig hoher Leistung erforderlich.

Aus letztgenanntem Grund wird in der Regel angestrebt, den optischen Übertragungsweg für die Strahlung vor allem innerhalb des Handstückes möglichst kurz zu halten. Eine diesbezügliche Option besteht in der Integration der Strahlungsquelle in das Handstück, was allerdings nachteiligerweise dazu führt, daß das Handstück verhältnismäßig groß und schwer wird. Ursachen der Gewichtszunahme sind dabei nicht zuletzt die zur Kühlung erforderlichen Maßnahmen. Weiterhin besteht bei integrierter Strahlungsquelle die Notwendigkeit, das Handstück über Energiezuführ- und Steuerleitungen zu versorgen, wodurch dessen Handhabung in unerwünschtem Maße behindert wird.

Werden als Laserstrahlungsquelle gewichtssparend Dioden verwendet und diese in das Handstück integriert, tritt nachteilig der Effekt auf, daß auf den Behandlungsort die einzelnen von Laserdioden erzeugten Laserbarren abgebildet werden und somit keine homogene Ausleuchtung des zu behandelnden Hautareals gewährleistet ist. Auch lassen sich hierbei die gewünschten Fleckgrößen nicht durch einfaches Wechseln eines optischen Elementes, wie etwa das Wechseln des Quarz- oder Saphirblokkes bei der thermischen Lichtquelle, realisieren, da diese hier nicht verwendet werden.

Eine alternative Möglichkeit zur integrierten Strahlungsquelle besteht darin, die elektromagnetische Strahlung in einer separaten, vom Handstück getrennten Strahlungsquelle zu erzeugen und diese mit dem Handstück über eine Lichtleiteinrichtung zu verbinden. Als Lichtleiteinrichtungen kommen Flüssigkeitslichtleiter oder auch Bündel aus festen Lichtleitern in Betracht. Solche Lichtleiteinrichtungen haben den Vorteil, daß sie Licht unter einem großen Winkel aufnehmen können und auch bei einem verhältnismäßig großen Querschnitt noch flexibel sind, so daß die nahezu ungehinderte Ausrichtung des Handstückes auf den zu behandelnden Hautabschnitt möglich ist.

Allerdings tritt die Strahlung, bedingt durch die große numerische Apertur solcher Lichtleiter, unter sehr großen Winkeln (typischerweise 67° bis 80°) aus diesen Lichtleitern wieder aus. Bei so großen Abstrahlwinkeln ist es schwierig, verschiedene definierte Fleckgrößen, etwa im Bereich von 5 bis 20 mm Durchmesser, am Applikationsort zu erzeugen, da zur Variation des Durchmessers die Abstandsänderung zwischen dem abstrahlungsseitigen Ende der Lichtleiteinrichtung und der Haut in einem Bereich von nur wenigen Millimetern vorgenommen werden muß.

Außerdem ändert sich mit zunehmendem Abstand des abstrahlungsseitigen Endes von der Haut nicht nur sehr schnell die Fleckgröße, sondern auch die Intensitätsverteilung innerhalb des Strahlquerschnittes. Besitzt beispielsweise ein Lichtleiter ein günstiges Verhältnis zwischen Durchmesser und Länge, liegt an seinem Ausgang ein Strahlprofil in Form eines "Flat-Top" vor. Mit zunehmendem Abstand von der Abstrahlfläche jedoch wird daraus eine Gauß-Verteilung. Ist die Intensität über den Strahlquerschnitt inhomogenen verteilt, kann es innerhalb des behandelten Hautareals zu Über- oder auch Unterbehandlungen kommen.

*In US-A-5,755,751 ist eine Einrichtung beschrieben, bei der während einer medizinischen oder kosmetischen Hautbehandlung Licht auf die Hautfläche abgestrahlt wird. Diese Einrichtung weist ein optisches Koppelelement auf, dessen Lichteintrittsfläche einer Lichtquelle zugewandt ist und dessen Lichtaustrittsfläche der Hautfläche zugewandt ist. Dieses Koppelelement ist aus einem Bündel von Lichtleitern gebildet, die in gegenseitiger Berührung miteinander stehen wobei optisch unwirksame Zwischenräume vermieden sind und die einerseits in der Lichteintrittsfläche und andererseits in der Lichtaustrittsfläche enden.* Somit offenbart US-A- 5,755,751 den Oberbegriff des Anspruchs 1.

### Beschreibung der Erfindung

Davon ausgehend liegt der Erfindung die Aufgabe zugrunde, die Mittel zur Strahlformung innerhalb eines Handstückes der eingangs beschriebenen Art derart zu verbessern, daß das Licht mit hoher Effizienz in die Haut eingebracht werden kann.

Erfindungsgemäß ist bei einem Handstück mit einem optischen Koppelelement, dessen Lichteintrittsfläche einer Lichtquelle zugewandt ist und dessen Lichtaustrittsfläche während der Hautbehandlung mit der Hautfläche in Kontakt steht, vorgesehen, daß das Koppelelement aus einem Bündel von Lichtleitern gebildet ist, die in der Lichteintrittsfläche einerseits und in der Lichtaustrittsfläche andererseits enden und die zumindest in ihren Endabschnitten in so enger seitlicher Berührung miteinander stehen, daß keine optisch unwirksamen Zwischenräume vorhanden sind und jeder der einzelner Lichtleiter innerhalb des kopplungselements an der Lichteintrittsfläche einen geringeren Querschnitt als ein der Lichtaustrittsfläche aufweist, so daß die Licht austrittsfläche gröβet ist als die Lichteintrittsfläche. Mit einem derartigen Koppelelement ist es vorteilhaft möglich, die Abbildung des abstrahlungsseitigen Endes einer Lichtleiteinrichtung bzw. einer Lichtquelle auf die Haut zu gewährleisten, ohne Maßnahmen zur Veränderung der Energieverteilung im Strahlquerschnitt treffen zu müssen. Im Gegensatz zur Verwendung beispielsweise eines Saphirblocks wie bisher im Stand der Technik, der aus einem homogenen Körper besteht, besteht das erfindungsgemäß für diese Anwendung vorgeschlagene Koppelelement aus einer Vielzahl von dünnen, zu einem massiven Stab verschmolzenen Einzelfasern, die innerhalb des Koppelelementes geordnet oder auch ungeordnet verlaufen können. Die dabei erzielbare Auflösung wird durch die Anzahl und den Querschnitt der Einzelfasern bestimmt.

Damit sind auf eine den Aufbau des Handstückes weitestgehend vereinfachende Weise die bisherigen Probleme der Wärmeentwicklung, der Strahlformung und auch der Baugröße gelöst. Es sei an dieser Stelle darauf hingewiesen, daß der Kontakt der Lichtaustrittsfläche mit der Hautfläche sowohl ein unmittelbarer direkter Kontakt als auch ein mittelbarer Kontakt sein kann, d.h. zwischen Lichtaustrittsfläche und Hautfläche kann sich beispielsweise auch ein dünner transparenter Körper, etwa eine Folie oder ein Plättchen, befinden.

Außerdem kann mit einem solchen Koppelelement auch die äußere geometrische Strahlform in einfacher Weise den Bedürfnissen der Behandlung angepaßt werden. So ist in einer Ausgestaltung der Erfindung vorgesehen, daß der Vinriß der Lichtaustrittsfläche von dem Umriß der Lichteintrittsfläche verschieden ist, wobei bevorzugt die Lichteintrittsfläche einen kreisrunden und die Lichtaustrittsfläche einen quadratischen Umriß hat. Damit ist einmal gewährleistet, daß der Übergang der Strahlung von der Lichtleiteinrichtung, die in der Regel einen kreisrunden Querschnitt aufweist, in die Lichteintrittsfläche (mit ebenfalls einem kreisrunden Querschnitt) weitestgehend ohne Verluste erfolgt.

Da die Lichtaustrittsfläche größer ist als die Lichteintrittsfläche, erfolgt eine vergrößerte Abbildung des austrittsseitigen Endes des Lichtleiters auf die Haut.

Dabei besteht der Vorteil, daß an der Lichtaustrittsfläche im wesentlichen dieselbe Strahlungsenergie und dieselbe Intensitätsverteilung anliegt wie an der Lichteintrittsfläche. Weist das Strahlprofil an der Lichteintrittsfläche eine Flat-Top-Form auf, so ist das auch an der Lichtaustrittsfläche der Fall. Wird also, der Kontakttechnik entsprechend, die Lichtaustrittsfläche auf die Haut aufgesetzt, wirkt die Strahlung mit homogen verteilter Intensität in die Haut ein.

Eine bevorzugte Ausgestaltung der Erfindung sieht vor, daß die Lichteintrittsfläche einen kreisrunden und die Lichtaustrittsfläche einen quadratischen Umriß hat.

Ein Koppelelement mit einer solchen Querschnittsänderung im Verlaufe seiner Übertragungslänge ist technologisch beispielsweise herstellbar, indem die Vielzahl der sehr dünnen Einzelfasern im Koppelelement zunächst zu einen massiven Stab verschmolzen werden, der dann in einem weiteren Fertigungsprozeß einer solchen Form angepaßt wird. Es entsteht dabei ein sich verjüngender Stab, der aus der Vielzahl einzelner, sich ebenfalls verjüngender Fasern besteht.

Es liegt im Rahmen der Erfindung, daß die Lichteintrittsfläche des Koppelelementes unmittelbar, das heißt ohne zwischengeschaltete Lichtübertragungseinrichtung, mit einer oder mehreren in das Handstück integrierten Lichtquellen in Verbindung steht, beispielsweise mit Laserdioden. Davon abweichend besteht jedoch eine bevorzugte Ausgestaltung der Erfindung darin, daß das Handstück über eine Lichtleiteinrichtung, die aus Flüssigleitern oder Fasern bestehen kann, mit einer Lichtquelle, bevorzugt einer Laserstrahlungsquelle, in Verbindung steht, wobei dem abstrahlungsseitigen Ende der Lichtleiteinrichtung innerhalb des Handstückes der Lichteintrittsfläche des Koppelelementes gegenübersteht. Die Übertragung erfolgt dann von einem kreisrunden Querschnitt am abstrahlungsseitigen Ende der Lichtleiteinrichtung auf eine kreisrunde Lichteintrittsfläche des Koppelelementes.

In einer ganz besonders Ausgestaltung weist die Umfangsfläche des Koppelelementes einen zwischen der Lichteintrittsfläche und der Lichtaustrittsfläche verlaufenden, zentrisch zur Mittenachse ausgerichteten kegelstumpfförmigen Abschnitt auf, dessen sich verjüngendes Ende zur Lichteintrittfläche hin gerichtet ist. Damit wird verhindert, daß das während der Behandlung von der Haut diffus rückgestreute oder reflektierte und dann entgegengesetzt zur Abstrahlungsrichtung durch die Lichtaustrittfläche hindurch wieder in das Koppelelement eintretende Laserlicht innerhalb des Koppelelementes bis zur Lichteintrittfläche weitergeleitet wird, erst dort wieder aus dem Koppelelement austritt und von Bauteilen des Handstückes in der Umgebung der Lichteintrittfläche absorbiert wird, was eine unerwünschte Erwärmung dieser Bauteile zur Folge hätte.

Dieser Erscheinung wirkt die erfindungsgemäß vorgeschlagene Ausgestaltung des kegelstumpfförmigen Abschnitt insofern entgegen, da nunmehr das Streu- bzw. Reflexionslicht nicht mehr innerhalb des Koppelelementes von der Lichtaustrittfläche bis zur Lichteintrittfläche weitergeleitet wird, sondern vor Erreichen der Lichteintrittfläche über die Umfangsfläche des kegelstumpfförmigen Abschnittes abgestrahlt wird. Dies erfolgt so weit vor der Lichteintrittsfläche, daß das Streu- bzw. Reflexionslicht von der Lichteintrittfläche und damit von der Einkoppelstelle für die zur Behandlung auf die Haut zu richtenden Laserstrahlung fern gehalten wird und dort somit auch die Wärmeentwicklung an dieser Stelle nicht in einem unzulässig hohen Maße eintreten kann.

In einer diesbezüglich noch weiter verbesserten Ausgestaltung ist der kegelstumpfförmige Abschnitt etwa bei dem halben Abstand zwischen der Lichteintrittsfläche und der Lichtaustrittsfläche vorgesehen, wobei die Umfangsflächen des Koppelelementes im Bereich zwischen der Lichteintrittsfläche und dem kleineren Durchmesser des kegelstumpfförmigen Abschnittes sowie zwischen dem größeren Durchmesser des kegelstumpfförmigen Abschnittes und der Lichtaustrittsfläche jeweils zylindrisch und konzentrisch zur Mittenachse verlaufend ausgebildet sind und wobei der Durchmesser der Lichteintrittsfläche dem kleineren Durchmesser des kegelstumpfförmigen Abschnittes und der Durchmesser der Lichtaustrittsfläche dem größeren Durchmesser der kegelstumpfförmigen Abschnittes entspricht.

Dabei kann der Umfang des Koppelelementes im Bereich zwischen der Lichteintrittsfläche und dem kleineren Durchmesser des kegelstumpfförmigen Abschnittes strekkenweise einen Durchmesser aufweisen, der größer ist als der Durchmesser der Lichteintrittsfläche bzw. größer ist als der kleinere Durchmesser des kegelstumpfförmigen Abschnittes, wobei jeweils zur Lichteintrittsfläche und zu dem kleineren Durchmesser des kegelstumpfförmigen Abschnittes hin Verjüngungen vorgesehen sind.

Selbstverständlich ist dabei die Umfangsfläche des Koppelelementes im Bereich des kegelstumpfförmigen Abschnittes für Licht transparent, das vom Inneren des Koppelelementes nach außen gerichtet ist.

Auf diese Weise sind die dem Stand der Technik bisher anhaftenden Nachteile, wie sie weiter oben dargelegt sind, aufgehoben.

### Kurze Beschreibung der Zeichnungen

Die Erfindung soll nachfolgend anhand eines Ausführungsbeispieles erläutert werden. Die zugehörigen Zeichnungen zeigen
- Fig.1: die Gesamtansicht eines erfinduncgsemäßen Handstückes mit einem integrierten Koppelelement,
- Fig.2: eine erste Ausführungsform eines Koppelelementes in einer Einzeldarstellung außerhalb des Handstückes mit einem Beispiel für die Abweichung der Querschnittsformen von Lichteintrittsfläche und Lichtaustrittsfläche,
- Fig.2a: das Koppelelement nach Fig.2 in der Ansicht A,
- Fig.2b: das Koppelelement nach Fig.2 in der Ansicht B,
- Fig.3: eine zweite Ausführungsform eines Koppelelementes in einer Einzeldarstellung außerhalb des Handstückes mit einem weiteren Beispiel für die Abweichung der Querschnittsformen von Lichteintrittsfläche und Lichtaustrittsfläche,
- Fig.3a: das Koppelelement nach Fig.3 in der Ansicht A,
- Fig.3b: das Koppelelement nach Fig.3 in der Ansicht B,
- Fig.4: ein Beispiel für die Änderung der Energieverteilung im Strahlquerschnitt mit zunehmender Entfernung vom abstrahlungsseitigen Ende eines Lichtleiters mit zylindrischem Querschnitt,
- Fig.5: ein Beispiel für die Änderung der Energieverteilung im Strahlquerschnitt mit zunehmendem Abstand von der Lichtaustrittsfläche eines kegelstumpfförmigen Koppelelementes,
- Fig.6: ein Koppelelement, dessen Lichteintrittsfläche innerhalb des Handstükkes mit Laserdioden in Verbindung steht,
- Fig.6a: ein Beispiel für die Strahlführung innerhalb der Einzelfasern, aus denen das Koppelelement gebildet ist,
- Fig.6b: ein Beispiel für das Erzielen der optimalen Energiedichte in der Wirktiefe der Strahlung unter der Hautoberfläche,
- Fig.7 bis Fig.9: verschiedene Ausgestaltungsformen des Koppelelementes.

### Ausführliche Beschreibung der Zeichnungen

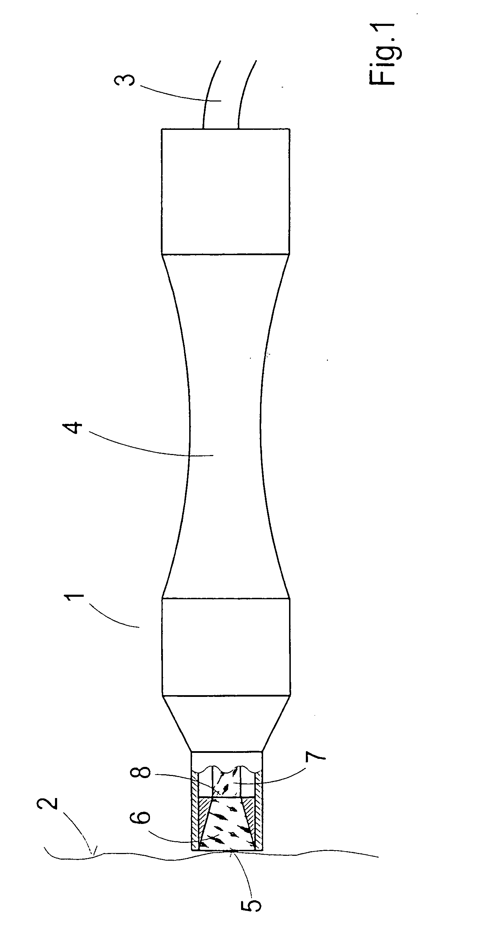

In Fig.1 ist ein Handstück 1 zur Abstrahlung von Licht auf eine Hautfläche 2, etwa zum Zwecke der Haarentfernung, dargestellt. Das Handstück 1 ist über eine Lichtleiteinrichtung 3, zum Beispiel einem Faserbündel, mit einer in der Zeichnung nicht dargestellten Strahlungsquelle verbunden. Als Strahlungsquelle sei ein Diodenlaser-Array vorgesehen.

Das Handstück weist eine Griffmulde 4 auf, die eine ergonomische Handhabung ermöglicht. Die Strahlungsenergie wird während der Behandlung über eine Lichtaustrittsfläche 5 in den zu behandelnden Hautabschnitt eingebracht.

Bei derartigen Behandlungen ist es erforderlich, daß die Strahlung mit einer über die gesamte Lichtaustrittsfläche 5 gleichmäßig verteilte Energiedichte in die Haut eingebracht wird. Es ist erstens dafür zu sorgen, daß die Ausdehnung der Lichtaustrittsfläche der zur Verfügung stehenden Laserleistung angemessen ist und zweitens die Energie entweder von der Lichtquelle bereits gleichmäßig über den Strahlquerschnitt verteilt bereitgestellt wird oder im Verlaufe des Übertragungsweges des Lichtes bis zur Lichtaustrittsfläche vergleichmäßigt wird. Oftmals ist es auch wünschenswert, daß die Lichtaustrittsfläche 5 einen bestimmten geometrischen Umriß, wie etwa ein Rechteck, ein Quadrat, ein Kreis o. ä. aufweist. Dabei hat insbesondere die quadratische Form den Vorteil, daß die Behandlung mehrerer einzelner Hautflächen lückenlos nebeneinander vorgenommen werden kann.

Es kommt also darauf an, die Strahlungsenergie möglichst verlustfrei von der Strahlungsquelle bis zur Lichtaustrittsfläche 5 zu transportieren, die Strahlformung so vorzunehmen, daß das Licht über die gesamte Lichtaustrittsfläche 5 mit gleichmäßiger Intensität abgestrahlt und dabei das abstrahlungsseitige Ende 7 der Lichtleiteinrichtung 3 vergrößert, auf die Hautfläche abgebildet wird. Diesbezüglich weist das Koppelelement 6 eine der Lichtaustrittsfläche 5 gegenüberliegende Lichteintrittsfläche 8 auf, die dem abstrahlungsseitigen Ende 7 der Lichtleiteinrichtung 3 zugewandt ist.

Erfindungsgemäß ist ein Koppelelement 6 vorgesehen, das aus einem Bündel von Lichtleitern gebildet ist. Diese Lichtleiter beginnen in der Lichteintrittsfläche 8 und enden in der Lichtaustrittsfläche 5, von wo die Strahlung in die Hautfläche 2 abgestrahlt wird. Dabei stehen die Lichtleiter innerhalb des Koppelelementes 6 zumindest an ihren Endabschnitten in so enger seitlicher Berührung miteinander, daß optisch unwirksame Zwischenräume weitestgehend ausgeschlossen bzw. im ldealfalle nicht vorhanden sind.

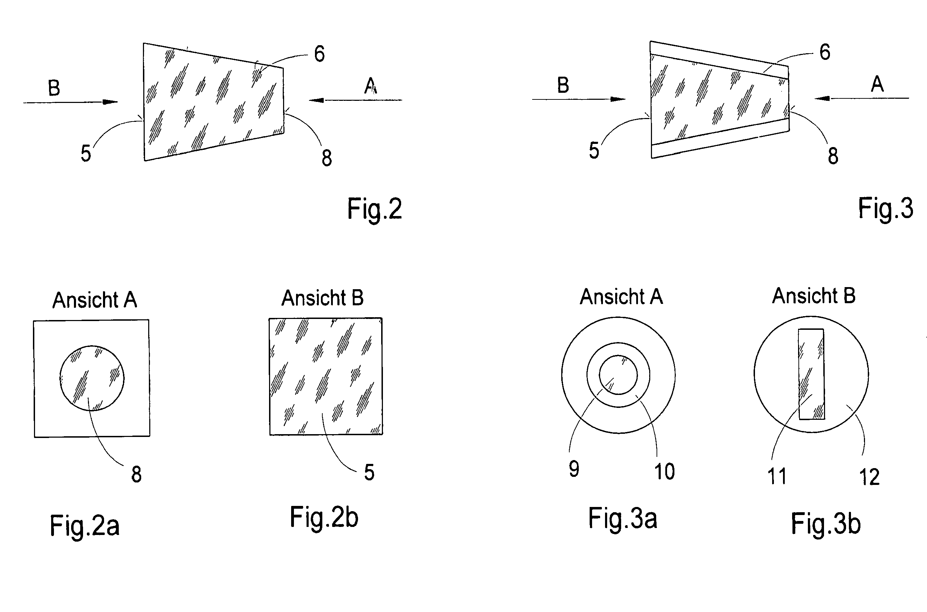

Mit anderen Worten, das Koppelelement 6 besteht erfindungsgemäß aus einer Vielzahl von zu einem massiven optischen Körper verschmolzenen sehr dünnen Einzelfasern. Die Formgebung für den Strahlquerschnitt erfolgt dadurch, daß die Lichtaustrittsfläche 5 eine größere Ausdehnung hat als die Lichteintrittsfläche 8, wodurch sich ein Koppelelement 6 wie in Fig.2 dargestellt ergibt. Hierbei weist jede der einzelnen Fasern innerhalb des Koppelelementes 6 an der Lichteintrittsfläche 8 einen geringeren Querschnitt auf als an der Lichtaustrittsfläche 5. Je größer die Anzahl der einzelnen Lichtleitfasern innerhalb des Koppelelementes 6 ist, um so größer ist die Auflösung, die mit dem Koppelelement bei der Abbildung des abstrahlungsseitigen Endes 7 der Lichtleiteinrichtung 3 erzielt werden kann.

Das in Fig.2 dargestellte Koppelelement 6 ist ein transparenter Körper mit einer kreisrunden Lichteintrittsfläche 8 und einer quadratischen Lichtaustrittsfläche 5. Dabei zeigen Fig.2a die Lichteintrittsfläche 8 in der Ansicht A und Fig.2b die Lichtaustrittsfläche 5 in der Ansicht B aus Fig.2. Auch hier sind die Fasern, aus denen der transparente Körper gebildet ist, miteinander verschmolzen und dabei so gezogen bzw. geformt, daß sich über die Länge zwischen der Lichteintrittsfläche 8 und der Lichtaustrittsfläche 5 hinweg die Querschnittsänderung von kreisrund auf quadratisch ergibt.

So wird vorteilhaft das am austrittseitigen Ende 7 der Lichtleiteinrichtung 3 verfügbare Licht bei geringsten Übergangsverlusten über die Lichteintrittsfläche 8 gleichmäßig zu dem für die Behandlung vorteilhaften und quadratischen Querschnitt der Lichtaustrittsfläche 5 übertragen.

Eine weitere Ausgestaltungsvariante des Koppelelementes 6 ist in Fig.3 dargestellt. Hier ist die Vielzahl von Lichtleitfasern, die zur Übertragung des Lichtes von der Lichteintrittsfläche 8 zur Lichtaustrittsfläche 5 dienen, von einem optisch nicht wirksamen Material umgeben, dessen äußere Form die Gestalt eines Kegelstumpfes hat. Aus Fig.3a, einer Ansicht A aus Fig.3, ist ersichtlich, daß die innerhalb des Kegelstumpfes verlaufenden Lichtleiter in der Lichteintrittsfläche 8 eine Kreisfläche 9 ausfüllen, die von einer Ringfläche 10 aus besagtem optisch nicht wirksamen Material umgeben ist.

Die Lichtaustrittsfläche 5 dieser Ausführungsform des Koppelelementes 6 ist in Fig.3b dargestellt, einer Ansicht B aus Fig.3. Hier enden die vielen Lichtleiter innerhalb einer Rechteckfläche 1 1 , die von einer optisch unwirksamen Fläche 12 umgeben ist. Auch hier sind die Lichtleiter innerhalb des Koppelelementes 6 so miteinander verschmolzen, daß sie an der Lichteintrittsfläche 8 und der Lichtaustrittsfläche 5 optisch wirksame Flächen unterschiedlicher Größe und Form ausfüllen. Auf diese Weise wird erreicht, daß an der Lichtaustrittsfläche 5 ein rechteckiger Spot zur Verfügung steht, dessen Energieverteilung durch die Lage der einzelnen Fasern und deren Verlauf von der Lichteintrittsfläche 8 bis zur Lichtaustrittsfläche 5 bestimmt ist.

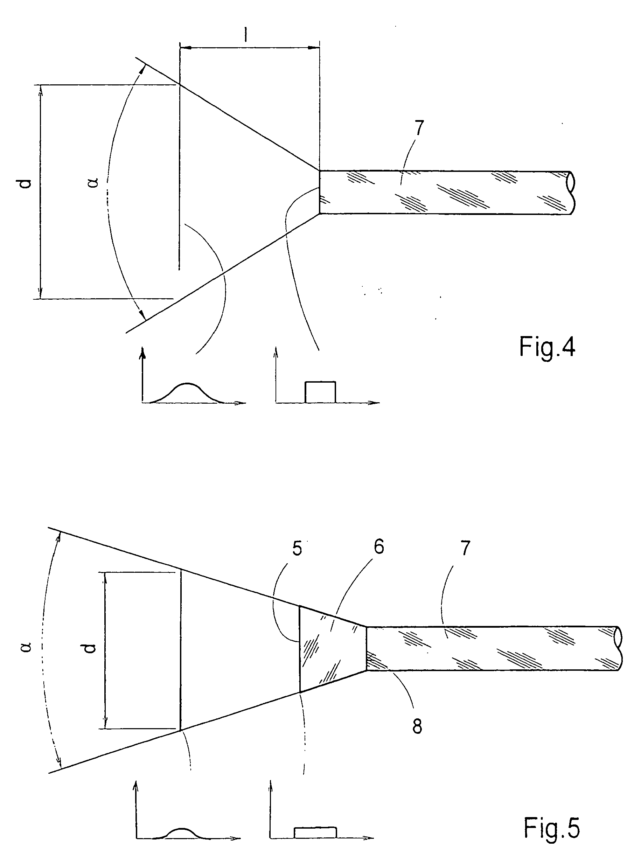

In Fig.4 ist ein Beispiel dafür dargestellt, wie sich Form und Energieverteilung innerhalb einer Laserstrahlung ändern, die vom abstrahlungsseitigen Ende 7 eines Lichtleiters in die freie Atmosphäre abgestrahlt wird. Dabei ist zu erkennen, daß sich das Licht in einem Kegel mit verhältnismäßig großem Kegelwinkel α ausbreitet, was zur Folge hat, daß sich erstens der Durchmesser d des Lichtfleckes mit zunehmendem Abstand I von der Lichtaustrittsfläche 5 ändert und zweitens auch die Energieverteilung im Lichtfleck mit dem Abstand I von der Energieverteilung an der Lichtaustrittsfläche 5 abweicht.

Es ist erkennbar, daß die Strahlung an der Lichtaustrittsfläche 5 mit einer Flat-Top-Form austritt, während die Energieverteilung im Abstand I von der Lichtaustrittsfläche 5 bereits eine Gauß-Form aufweist. Würde dieses abstrahlungsseitige Ende 7 unmittelbar auf die Haut aufgesetzt, würde eine Strahlung, die eine über den gesamten Querschnitt gleichmäßige Energieverteilung hat, mit sehr hoher Intensität in eine verhältnismäßig kleine Hautfläche eingebracht.

In Fig.5 dagegen ist dem abstrahlungsseitigen Ende 7 das Koppelelement 6 nachgeordnet, wobei mit dem Koppelelement 6 erreicht wird, daß die hohe Strahlungsleistung bei kleinem Strahlquerschnitt auf eine an der Lichtaustrittsfläche 5 zur Verfügung stehende Strahlung mit größerem Strahlquerschnitt, dafür aber geringerer Energiedichte und immer noch gleichmäßiger Energieverteilung zu Verfügung steht, woraus sich vorteilhaft ergibt, daß ein größeres Hautareal schonend und gleichmä-βig behandelt werden kann.

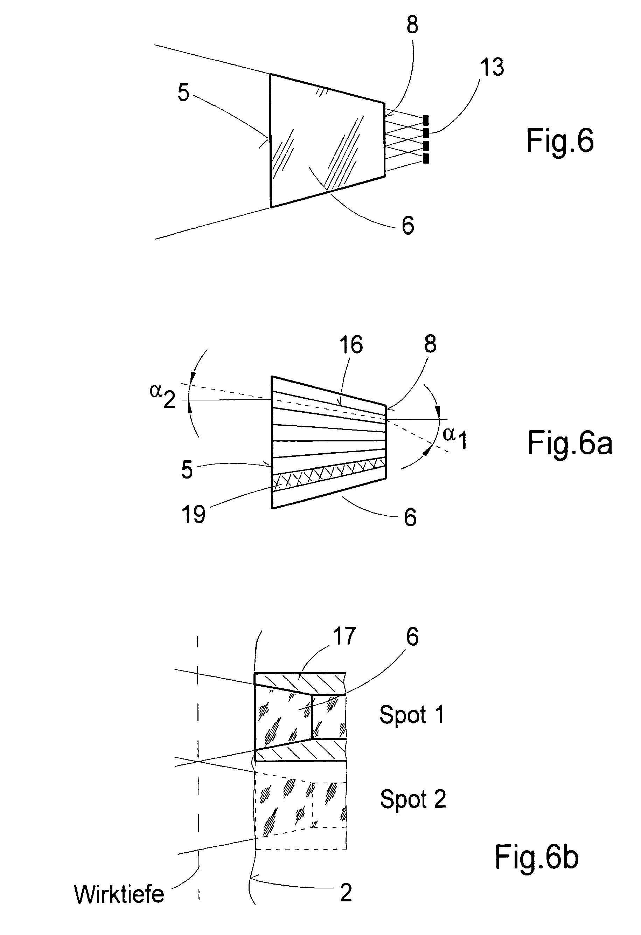

Fig.6 zeigt ein Beispiel, bei dem das Licht nicht von einem Ende 7 einer Lichtleiteinrichtung 3 in die Lichteintrittsfläche 8 des Koppelelementes 6 eingestrahlt wird, sondern von einer Quelle, die beispielsweise aus einem Array von Laserdioden 13 gebildet ist. Hierbei können die Enden der Lichtleiter in der Lichteintrittsfläche 8 so positioniert sein, daß sie einerseits das Licht der einzelnen Laserdioden 13 optimal aufnehmen und andererseits an der Lichtaustrittsfläche 5 vergleichmäßigt abgeben, wobei die anordnungsbedingten Hell-Dunkel-Übergänge innerhalb des Laserdiodenarrays mit dem Verlauf der Lichtleiter innerhalb des Koppelelementes 6 ausgeglichen werden können.

Bei Verwendung eines Koppelelementes nach Fig.5 und Fig.6 werden vorteilhafte Effekte im Hinblick auf die Wirkung der Strahlung in der Tiefe unter der Hautfläche 2 erzielt, wie nachfolgend anhand Fig.6a und Fig.6b dargestellt ist.

In Fig.6a ist das Prinzip der Strahlungsreflexion innerhalb des Koppelelementes 6 dargestellt. Es sei angenommen, das Koppelelement 6 besitze die Form eines Kegelstumpfes. Dann erfolgt die Reflexion an den Mantelflächen 16 innerhalb aller Einzelfasern 19 des Koppelelementes 6 wie aus Fig.6a ersichtlich: der Austrittswinkel α₂ eines Strahlungsanteiles ist kleiner als der Eintrittswinkel α₁ desselben Strahlungsanteiles. Dabei ist das Verhältnis von Austrittswinkel α₂ zu Eintrittswinkel α₁ umgekehrt proportional zum Verhältnis von Lichteintrittsfläche 8 zur Lichtaustrittsfläche 5. Die Einzelfasern 19 innerhalb des Koppelelementes 6 sind hier der Übersichtlichkeit wegen vergrößert dargestellt.

Mit anderen Worten: Auf der Lichtaustrittseite des Koppelelementes 6 hat die Strahlung eine deutlich geringere Divergenz als auf der Lichteintrittseite. Damit wird in einer für eine erfolgreiche Behandlung notwendigen Wirktiefe unter der Hautfläche 2 eine vorteilhafte Energiedichte erzielt, denn es lassen sich Überlappungen von nebeneinander aufgesetzten Spots und damit unerwünschte Überdosierungen in Hautbereichen vermeiden.

Dies ist beispielhaft in Fig.6b dargestellt. Hier sind die Außenabmessungen des Koppelelementes 6 und die Divergenz der in die Haut eindringenden Strahlung so bemessen, daß beim Nebeneinandersetzen mehrerer Spots in der Wirktiefe keine Überlappung der Strahlung erfolgt. So kann die Ausdehnung einer optisch nicht aktiven, das Koppelelement 6 umschließenden Hülle 17 in bezug auf die Divergenz so bemessen sein, daß beim Aufsetzen der Hülle 17 auf zwei lückenlos benachbarte Abschnitte der Hautfläche 2 von Spot 1 zu Spot 2 eine optimale Energiedichte in der Wirktiefe erzielt wird - ohne Überlappung mit der Folge von Überdosierungen und auch ohne Fehlstellen mit der Folge unzureichender Behandlung.

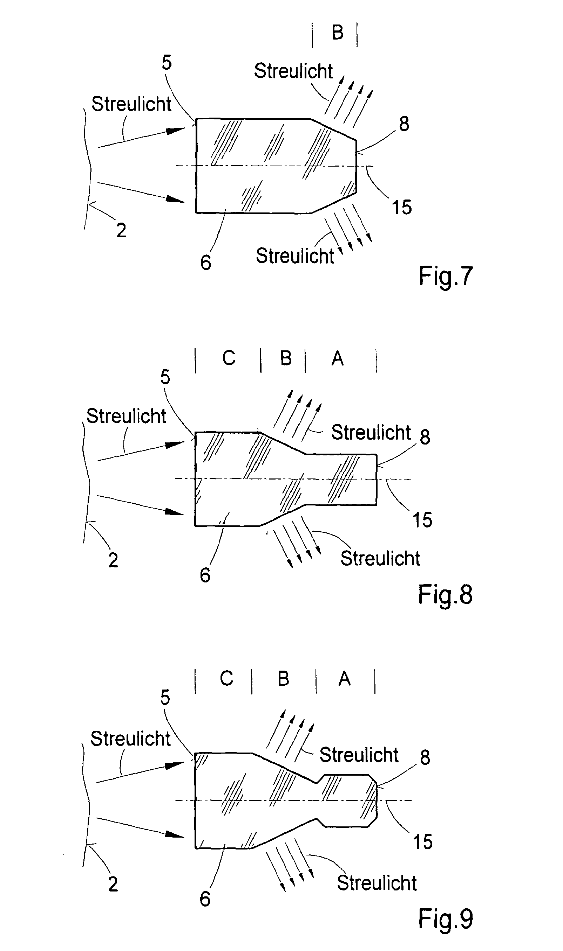

In Fig.7, Fig.8 und Fig.9 sind weitere Beispiele für vorteilhafte Ausgestaltungen des Koppelelementes 6 dargestellt.

So weist das Koppelelement in der Ausgestaltung gemäß Fig.7 an seiner Umfangsfläche im Bereich B zwischen der Lichteintrittsfläche 8 und der Lichtaustrittsfläche 5 einen kegelstumpfförmigen Abschnitt auf, der zentrisch zur Mittenachse 1 5 verläuft und dessen sich verjüngendes Ende zur Lichteintrittsfläche 8 hin gerichtet ist. Auf diese Weise wird dafür gesorgt, daß während der Behandlung von der Hautfläche 2 kommendes, entgegengesetzt zu der auf die Hautfläche 2 gerichteten Laserstrahlung durch die Lichtaustrittsfläche 5 wieder in das Koppelelement 6 eintretendes Streu- oder Reflexionslicht an der Umfangsfläche des kegelstumpfförmigen Abschnittes wieder aus dem Koppelelement 6 austritt. Damit wird verhindert, daß das Streu- bzw. Reflexionslicht bis zur Lichteintrittsfläche 8 gelangt und erst durch diese hindurch wieder aus dem Koppelelement 6 austritt, was eine Absorption dieser Streu- bzw. Reflexionslichtstrahlung durch in der näheren Umgebung der Lichteintrittsfläche 8 befindliche mechanische Bauteile und damit deren Erwärmung zur Folge hätte.

In einer weiterführenden Ausgestaltung nach Fig.8 ist der kegelstumpfförmige Abschnitt etwa im halben Abstand zwischen der Lichteintrittsfläche 8 und der Lichtaustrittsfläche 5 angeordnet. Hiermit wird ebenfalls erreicht, daß das durch die Lichtaustrittsfläche 5 unerwünscht eintretende Streu- bzw. Reflexionslicht an den Umfangsflächen des kegelstumpfförmigen Abschnittes austritt, wobei hier vorteilhafterweise der Bereich B mit dem kegelstumpfförmigen Abschnitt weiter von der Lichteintrittfläche 8 entfernt ist als in der Darstellung nach Fig.7.

Bei dieser Ausgestaltung kann vorgesehen sein, daß sowohl die Lichteintrittfläche 8 als auch die Lichtaustrittsfläche 5 jeweils kreisrund sind, wobei der Bereich A, der sich zwischen Lichteintrittfläche 8 bis zum Beginn des kegelstumpfförmigen Abschnittes erstreckt, zylindrisch ausgeführt ist. Dasselbe trifft zu für den Bereich C, der bei dem kegelstumpfförmigen Abschnitt beginnt und sich bis zu der Lichtaustrittfläche 5 erstreckt.

Selbstverständlich kann hierbei vorgesehen sein, daß die Lichtaustrittfläche 5 wie bereits weiter oben beschrieben eine quadratisch oder auch anderweitig geformten Umriß aufweist, wobei sich dann die Querschnittsform von der Kreisform im Bereich des Übergangs zwischen den Bereichen B und C bis zur Lichtaustrittsfläche 5 hin in die für die Lichtaustrittfläche 5 vorgegebene Form ändert.

Eine besonders bevorzugte Ausgestaltung des Koppelelementes 6 ist in Fig. 9 dargestellt. Hier weist der Umfang des Koppelelementes 6 im Bereich zwischen der Lichteintrittsfläche 8 und dem kleineren Durchmesser d₁ des kegelstumpfförmigen Abschnittes streckenweise einen Durchmesser, der größer ist als der Durchmesser der Lichteintrittsfläche 8 und auch größer ist als der kleinere Durchmesser d₁ des kegelstumpfförmigen Abschnittes. Bei dieser Ausbildung des Koppelelementes 6 liegt der kegelstumpfförmige Abschnitt ebenso wie bei der Ausgestaltung nach Fig.8 verhältnismäßig weit von der Lichteintrittsfläche 8 entfernt, so daß das vom Inneren des Koppelelementes 6 nach außen abgestrahlte Streu- bzw. Reflexionslicht von der Lichteintrittfläche 8 ferngehalten wird. So wird insbesondere mit der Ausbildung nach Fig.9 effektiv erreicht, daß das Streu- bzw. Reflexionslicht nicht bis zur Lichteintrittsfläche 8 gelangt.

Das Koppelelement 6 bewirkt bei der Abstrahlung der auf die Haut gerichteten Laserstrahlung eine Vergrößerung, die dem Verhältnis von Lichtaustrittsfläche 5 zu Lichteintrittsfläche 8 entspricht. Wird der kleiner Durchmesser d₁ des kegelstumpfförmigen Abschnittes bei voller Ausnutzung der Numerischen Apertur des Koppelelementes beispielsweise gleich dem Durchmesser der Lichteintrittsfläche 8 gewählt, so besteht bezüglich der durch die Lichteintrittsfläche 8 in das Koppelelement 6 eingestrahlten Laserstrahlung durch die Einengung beim kleinen Durchmesser d₁ kein Transmissionsverlust. Daraus folgt, daß der Durchmesser d₁ auch kleiner sein kann als der Durchmesser der Lichteintrittsfläche 8, sofern für die Laserstrahlung nicht die volle Numerische Apertur des Koppelelementes 6 ausgenutzt werden soll. Streu- bzw. Reflexionslicht, das unter einem Winkel α durch die Lichtaustrittsfläche 5 in das Koppelelement 6 eintritt, der größer ist als es die Numerische Apertur bzw. der Vergrößerungsfaktor zuläßt, wird an dem kegelstumpfförmigen Abschnitt ausgekoppelt bzw. nach außen abgestrahlt.

## Patentansprüche

1. Handstück zur Abstrahlung von Licht auf eine Hautfläche bei einer medizinischen oder kosmetischen Hautbehandlung, wobei im Handstück (1) ein optisches Koppelelement (6) vorhanden ist, dessen Lichteintrittsfläche (8) einer Lichtquelle zugewandt ist und dessen Lichtaustrittsfläche (5) während der Hautbehandlung mit der Hautfläche (2) in Kontakt steht, und
- das Koppelelement (6) aus einem Bündel von Lichtleitern gebildet ist, die in der Lichteintrittsfläche (8) einerseits und in der Lichtaustrittsfläche (5) andererseits enden und die zumindest in ihren Endabschnitten in so enger seitlicher Berührung miteinander stehen, daß optisch unwirksame Zwischenräume vermieden sind, **dadurch gekennzeichnet, daß**
- jeder der einzelnen Lichtleiter innerhalb des Kopplungselements (6) an der Lichteintrittsfläche (8) einen geringeren Querschnitt als an der Lichtaustrittsfläche (5) aufweist, so daß
- die Lichtaustrittsfläche (5) größer ist als die Lichteintrittsfläche (8).

2. Handstück nach Anspruch 1 , **dadurch gekennzeichnet, daß** die Lichtaustrittsfläche (5) und die Lichteintrittsfläche (8) verschiedene geometrische Umrisse aufweisen.

3. Handstück nach einem der vorgenannten Ansprüche, **dadurch gekennzeichnet, daß** die Umfangsfläche des Koppelelementes (6) einen zwischen der Lichteintrittsfläche (8) und der Lichtaustrittsfläche (5) verlaufenden, zentrisch zur Mittenachse (15) ausgerichteten kegelstumpfförmigen Abschnitt aufweist, dessen sich verjüngendes Ende zur Lichteintrittsfläche (8) hin gerichtet ist.

4. Handstück nach Anspruch 3, **dadurch gekennzeichnet, daß** der kegelstumpfförmige Abschnitt im wesentlichen bei dem halbem Abstand zwischen der Lichteintrittsfläche (8) und der Lichtaustrittsfläche (5) vorgesehen ist, wobei die Umfangsflächen des Koppelelementes (6) im Bereich zwischen der Lichteintrittsfläche (8) und dem kleineren Durchmesser des kegelstumpfförmigen Abschnittes sowie zwischen dem größeren Durchmesser des kegelstumpfförmigen Abschnittes und der Lichtaustrittsfläche (5) jeweils zylindrisch und konzentrisch zur Mittenachse (15) verlaufend ausgebildet sind, und wobei der Durchmesser der Lichteintrittsfläche (8) dem kleineren Durchmesser des kegelstumpfförmigen Abschnittes und der Durchmesser der Lichtaustrittsfläche (5) dem größeren Durchmesser des kegelstumpfförmigen Abschnittes entspricht.

5. Handstück nach Anspruch 4, **dadurch gekennzeichnet, daß** der Umfang des Koppelelementes (6) im Bereich zwischen der Lichteintrittsfläche (8) und dem kleineren Durchmesser des kegelstumpfförmigen Abschnittes streckenweise einen Durchmesser aufweist, der größer ist als der Durchmesser der Lichteintrittfläche (8) bzw. größer ist als der kleinere Durchmesser des kegelstumpfförmigen Abschnittes, wobei jeweils zur Lichteintrittsfläche (8) und zu dem kleineren Durchmesser des kegelstumpfförmigen Abschnittes hin Verjüngungen vorgesehen sind.

6. Handstück nach einem der Ansprüche 3 bis 5, **dadurch gekennzeichnet, daß** die Umfangsfläche des Koppelelementes (6) im Bereich des kegelstumpfförmigen Abschnittes für Licht transparent ist, das vom Inneren des Koppelelementes (6) nach außen gerichtet ist.

7. Handstück nach einem der vorgenannten Ansprüche, **dadurch gekennzeichnet, daß** die Lichteintrittsfläche (8) einen kreisrunden und die Lichtaustrittsfläche (5) einen quadratischen Umriß hat.

8. Handstück nach einem der vorgenannten Ansprüche, **dadurch gekennzeichnet, daß** das Handstück (1) über eine Lichtleiteinrichtung (3) mit einer Lichtquelle, bevorzugt einer Laserstrahlungsquelle, in Verbindung steht und das abstrahlungsseitige Ende (7) der Lichtleiteinrichtung (3) innerhalb des Handstückes (1) der Lichteintrittsfläche (8) gegenübersteht.

9. Handstück nach einem der Ansprüche 1 bis 7, **dadurch gekennzeichnet, daß** die Lichtquelle mindestens eine Laserdiode (13) umfaßt und in das Handstück (1) integriert ist.

## Claims

1. Hand piece for radiating light onto a skin surface during a medical or cosmetic skin treatment, wherein in the hand piece (1) an optical coupling element (6) is provided in which the light entry surface (8) faces a light source and the light exit surface (5) is in contact with the skin surface (2) during the skin treatment, and
- the coupling element (6) is formed by a bundle of light guides which end in the light entry surface (8) on the one hand and in the light exit surface (5) on the other hand and which are in such close lateral contact with each other at least in their end sections that optically inactive intermediate spaces are avoided, **characterised in that**
- each of the individual light guides within the coupling element (6) has a cross-section on the light entry surface (8) than on the light exit surface (5), so that
- the light exit surface (5) is larger than the light entry surface (8).

2. Hand piece as claimed in Claim 1, **characterised in that** the light exit surface (5) and the light entry surface (8) have different geometrical outlines.

3. Hand piece as claimed in any one of the preceding claims, **characterised in that** the peripheral surface of the coupling element (6) has a section in the shape of a truncated cone which extends between the light entry surface (8) and the light exit surface (5) and is aligned centrally with respect to the centre axis (15) and of which the tapering end is directed towards the light entry surface (8).

4. Hand piece as claimed in Claim 3, **characterised in that** the section in the shape of a truncated cone is provided at substantially half the distance between the light entry surface (8) and the light exit surface (5), wherein the peripheral surfaces of the coupling element (6) are constructed so that in each case they extend cylindrically and concentrically with respect to the centre axis (15) in the region between the light entry surface (8) and the smaller diameter of the section in the shape of a truncated cone and between the larger diameter of the section in the shape of a truncated cone and the light exit surface (5), and wherein the diameter of the light entry surface (8) corresponds to the smaller diameter of the section in the shape of a truncated cone and the diameter of the light exit surface (5) corresponds to the larger diameter of the section in the shape of a truncated cone.

5. Hand piece as claimed in Claim 4, **characterised in that** in the region between the light entry surface (8) and the smaller diameter of the section in the shape of a truncated cone the circumference of the coupling element (6) has in some sections a diameter which is larger than the diameter of the light entry surface (8) or larger than the smaller diameter of the section in the shape of a truncated cone, wherein in each case tapers are provided towards the light entry surface (8) and towards the smaller diameter of the section in the shape of a truncated cone.

6. Hand piece as claimed in any one of Claims 3 to 5, **characterised in that** the peripheral surface of the coupling element (6) in the region of the section in the shape of a truncated cone is transparent to light which is directed outwards from the interior of the coupling element (6).

7. Hand piece as claimed in any one of the preceding claims, **characterised in that** the light entry surface (8) has a circular outline and the light exit surface (5) has a square outline.

8. Hand piece as claimed in any one of the preceding claims, **characterised in that** the hand piece (1) is connected to a light source, preferably a laser light source, by way of a light guide device (3) and end (7) of the light guide device (3) on the emission side is opposite the light entry surface (8) within the hand piece (1).

9. Hand piece as claimed in any one of Claims 1 to 7, **characterised in that** the light source comprises at least one laser diode (13) and is integrated into the hand piece (1).

## Revendications

1. Pièce à main pour le rayonnement de lumière sur une surface cutanée pour un traitement cutané médical ou cosmétique, sachant que dans la pièce à main (1) se trouve un élément de couplage optique (6) dont la surface d'entrée de la lumière (8) est tournée vers une source de lumière et dont la surface de sortie de la lumière (5) est en contact avec la surface de la peau (2) pendant le traitement cutané, et
que l'élément de couplage (6) est formé d'un faisceau de conducteurs optiques qui finissent dans la surface d'entrée de la lumière (8) d'un côté et dans la surface de sortie de la lumière (5) de l'autre côté, et qui se trouvent au moins dans leurs sections d'extrémité en contact latéral si étroit les uns avec les autres que des espaces intermédiaires optiquement inefficaces sont évités, **caractérisée en ce que**
chacun des différents conducteurs optiques à l'intérieur de l'élément de couplage (6) présente à la surface d'entrée de la lumière (8) une section plus petite qu'à la surface de sortie de la lumière (5), de telle sorte que
la surface de sortie de la lumière (5) est plus grande que la surface d'entrée de la lumière (8).

2. Pièce à main selon la revendication 1, **caractérisée en ce que** la surface de sortie de la lumière (5) et la surface d'entrée de la lumière (8) présentent des contours géométriques différents.

3. Pièce à main selon l'une des revendications précédentes, **caractérisée en ce que** la surface périphérique de l'élément de couplage (6) comporte un tronçon tronconique orienté concentriquement par rapport à l'axe médian (15), s'étendant entre la surface d'entrée de la lumière (8) et la surface de sortie de la lumière (5), dont l'extrémité effilée est tournée vers la surface d'entrée de la lumière (8).

4. Pièce à main selon la revendication 3, **caractérisée en ce que** le tronçon tronconique est prévu pour l'essentiel à la moitié de la distance entre la surface d'entrée de la lumière (8) et la surface de sortie de la lumière (5), sachant que les surfaces périphériques de l'élément de couplage (6) dans la zone entre la surface d'entrée de la lumière (8) et le plus petit diamètre du tronçon tronconique, ainsi qu'entre le plus grand diamètre du tronçon tronconique et la surface de sortie de la lumière (5) sont configurés de sorte à s'étendre respectivement cylindriquement et concentriquement par rapport à l'axe médian (15), et sachant que le diamètre de la surface d'entrée de la lumière (8) correspond au plus petit diamètre du tronçon tronconique et que le diamètre de la surface de sortie de la lumière (5) correspond au plus grand diamètre du tronçon tronconique.

5. Pièce à main selon la revendication 4, **caractérisée en ce que** la périphérie de l'élément de couplage (6) présente par endroits dans la zone entre la surface d'entrée de la lumière (8) et le plus petit diamètre du tronçon tronconique un diamètre qui est plus grand que le diamètre de la surface d'entrée de la lumière (8), respectivement plus grand que le plus petit diamètre du tronçon tronconique, sachant que des rétrécissements sont prévus respectivement vers la surface d'entrée de la lumière (8) et vers le plus petit diamètre du tronçon tronconique.

6. Pièce à main selon l'une des revendications 3 à 5, **caractérisée en ce que**, dans la zone du tronçon tronconique, la surface périphérique de l'élément de couplage (6) est transparente à la lumière qui est dirigée de l'intérieur de l'élément de couplage (6) vers l'extérieur.

7. Pièce à main selon l'une des revendications précédentes, **caractérisée en ce que** la surface d'entrée de la lumière (8) a un contour circulaire et **en ce que** la surface de sortie de la lumière (5) a un contour carré.

8. Pièce à main selon l'une des revendications précédentes, **caractérisée en ce que** la pièce à main (1) se trouve en liaison par un dispositif conducteur optique (3) avec une source de lumière, de préférence une source de rayonnement laser, et que l'extrémité de sortie du rayonnement (7) du dispositif conducteur optique (3) se trouve à l'intérieur de la pièce à main (1) en face de la surface d'entrée de la lumière (8).

9. Pièce à main selon l'une des revendications 1 à 7, **caractérisée en ce que** la source de lumière comprend au moins une diode laser (13) et est intégrée dans la pièce à main (1).
